# EUROPEAN PATENT APPLICATION

(11) **EP 1 184 030 A1**
(43) Date of publication of application: **06.03.2002**
(21) Application number: 01810766.4
(22) Date of filing: 09.08.2001
(51) Int. Cl.: A61K 7/32, A61K 7/46, A01N 31/06

(54) **Antibacterial composition comprising trimethylnorbornanylcyclohexanol derivatives**

(30) Priority: 14.08.2000 EP 00117496
(71) Applicant: Givaudan SA, 1214 Vernier (CH)
(72) Inventor: Natsch, Andreas, 8707 Uetikon (CH)
(74) Representative: Patentanwälte Schaad, Balass, Menzl & Partner AG

(57) **Abstract**

The present invention relates to an antibacterial composition comprising one or more compound(s) of formula I 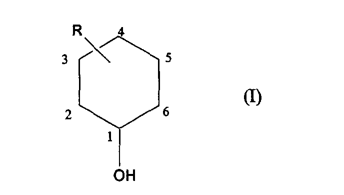 as an active component, and at least one other ingredient, wherein formula I R is a residue of the formula II 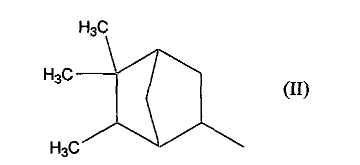 at the position 2, 3, 4 or 5 and
if R is at the position 5, a hydrogen in position 2 is substituted by a methoxy group, and
if R is at the position 4, a hydrogen in position 2 may optionally be substituted by a methoxy group.

The antibacterial composition is active against *Corynebacteria, Staphylococci,* and *Brevibacteria.* Therefore it can inhibit formation of different kinds of body malodor.

## Description

The present invention relates to an antibacterial composition.

Body malodor is formed when fresh perspiration, which is odorless per se, is decomposed by bacteria such as *Staphylococci* and *Corynebacteria,* both genera belonging to the gram-positive *Eubacteriaceae.*

Cosmetic deodorants are intended to inhibit formation of axillary body malodor. They are based on different active principles. One principle is the suppression of perspiration by astringents. According to a second principle the bacterial flora on the skin is reduced by antibacterial substances. But many commercially used antibacterial compounds affect the entire microbial flora on the skin. In addition some agents have higher activity against the low-odor forming *Staphylococci* bacteria than against the *Corynebacteria* and may thus favour the latter, which is highly undesirable.

Foot deodorants and deodorants for shoes are used to prevent the formation of a typical cheesy smell of feet. This odor is formed by *Brevibacteria* under prolonged periods of humidity which may occur especially in individuals with excessive transpiration or in shoes with insufficient aeration.

Another skin manifestation attributed to a bacterial origin is acne, which is commonly treated with topically applied cosmetic products containing antibacterial agents.

Antibacterial compounds currently used in deodorant or antiperspirant products include for example Triclosan (2,4,4'-trichloro-2'-hydroxy-diphenyl-ether). However, the use of such chlorinated compounds as antibacterial agents are strongly questioned by consumer organisations.

Antibacterial properties of certain odoriferous substances, essential oils or other fragrance ingredients are known to be used to manufacture deodorising fragrance compositions. One natural fragrance compound with particularly high activity is 3,7,11-trimethyl-2,6,10-dodecatrien-1-ol (Farnesol). Its use in deodorising fragrance compositions has been described in DE 27 28 921 B2 and DE 33 15 058 Al. However, to obtain its antibacterial effect, higher levels than the ones used in customary perfumery need to be employed.

Another natural fragrance ingredient known for its antibacterial activity is Sandalwood oil. In a recent study by Viollon et Chaumont (Parfums & cosmetiques, 1994, 116:67-70) the antibacterial activity against axilla bacteria of 26 essential oils were compared. Among them Sandalwood oil has the strongest activity. However, high price and limited availability of this natural oil hamper its widespread use in commercial cosmetic products at levels needed for a growth inhibition of skin bacteria.

It is an object of the present invention to provide a composition having an antibacterial effect against *Corynebacteria.*

Further it is an object of the present invention to provide a composition having an antibacterial effect against *Staphylococci.*

Further it is an object of the present invention to provide a composition having an antibacterial effect against *Brevibacteria.*

Further it is an object of the present invention to provide a composition having an antibacterial effect against *Propionibacteria.*

The present invention is based on the surprising finding that among 250 commonly used fragrance raw materials an antibacterial composition comprising one or more compound(s) of formula I 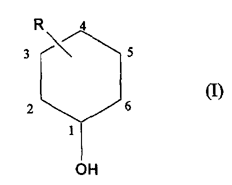 as an active component, and at least one other ingredient,
wherein formula I R is a residue of the formula II 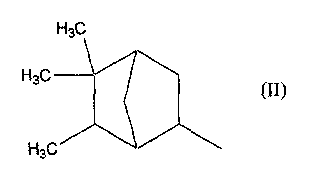 at the position 2, 3, 4 or 5 and
if R is at the position 5, a hydrogen in position 2 is substituted by a methoxy group, and
if R is at the position 4, a hydrogen in position 2 may optionally be substituted by a methoxy group,
is very active against a collection of *Corynebacterium sp.* strains isolated from healthy human volunteers with an activity even slightly higher than Sandalwood oil, and only slightly below the one of Farnesol. The activity of the active component is significantly higher than e.g. the activity of Sandalore and Radjanol. It was shown that the compounds according to the present invention offer both, outstanding olfactive properties as well as an excellent antibacterial activity. Furthermore the intensity of the antibacterial effect against *Corynebacteria* and *Staphylococcus* is equal, which is not the case for Triclosan. The latter compound is much more active for the low-odor forming *Staphylococci* then for the *Corynebacteria,* and application of low amounts of this compound may thus favour populations of *Corynehacteria.*

The colourless viscous liquid Sandela is obtained by a two step reaction, wherein Camphene and Guaiacol are treated with an acidic catalyst followed by hydrogenation (US patent 3'499'937) and its preparation is relatively cheap. The above two step reaction results in a mixture of the compounds of formula I, i.e. 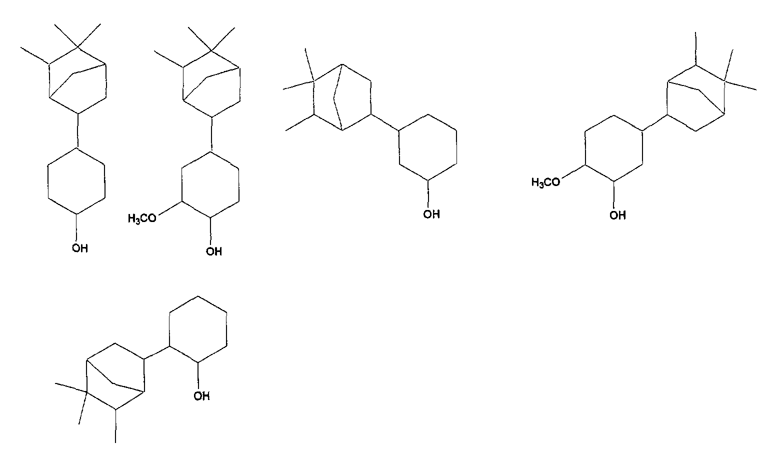 and all possible enantiomers. Every compound of the formula I can be isolated by destillation or chromatography. Said compounds may be used alone or in combination with each other as the active component.

The highest activity against *Corynebacteria* was found for 2-methoxy-4-(5,5,6-trimethylbicyclo[2.2.1]hept-2-yl)cyclohexan-1-ol and 2-methoxy-5-(5,5,6-trimethyl-bicyclo[2.2.1]hept-2-yl)cyclohexan-1-ol. Especially with 2-methoxy-4-(5,5,6-trimethylbicyclo[2.2.1]hept-2-yl)cyclohexan-1-ol an outstanding activity is achieved.

Another preferred embodiment of the composition according to the present invention is a composition, wherein the active component is 3-(5,5,6-trimethylbicyclo[2.2.1]hept-2-yl)cyclohexan-1-ol).

Further, it was shown, that the active component in the composition according to the present invention has also strong antibacterial properties against *Brevibacterium epidermidis*, the organism involved in formation of foot odor. The active component in the composition according to the present invention is an appropriate synthetic molecule to control this organism. Their activity is equal as natural Sandalwood oil and 2 to 6 times higher than the synthetics Radjanol and Sandalore. Therefore the active component in the composition according to the present invention can inhibit formation of different kinds of body malodor.

Furthermore it was shown, that active component in the composition according to the present invention has good antibacterial properties against *Propionibacterium acnes* as well. Therefore it is useful for prevention and treatment of acne.

Odoriferous substances are by definition volatile compounds which evaporate from the skin and therefore loose their deodorising effect. Furthermore small water-soluble fragrance compounds can get dissolved in sweat and are thus rapidly diluted under conditions of high sweat secretion which further reduces the duration of the deodorising effect. Therefore a low water solubility indicated by a high LogP value and a low volatility indicated by low vapour pressure are desired for odoriferous substances which are used for their antibacterial activity. Indeed it has been found that 2-(5,5,6-trimethylbicyclo[2.2.1]hept-2-yl)-cyclohexanol, 3-(5,5,6-trimethylbicyclo[2.2.1]hept-2-yl)-cyclohexanol, and 4-(5,5,6-trimethylbicyclo[2.2.1]hept-2-yl)-cyclohexanol, besides exhibiting pronounced antibacterial activity, meet these criteria. They have a vapour pressure of about 2.2 µg/L and a calculated LogP of about 5.27. Regarding this they are similar to Farnesol, which has a vapour pressure of 1.35 µg/L and a calculated LogP value of 5.31. Compared to e.g. the common antibacterial perfume ingredient Eugenol with a vapour pressure of 109 µg/L and a calculated LogP of 2.2 their deodorant activity is therefore maintained over a prolonged period of time when it is applied to the skin.

Since products which exert an antibacterial activity by means of individual fragrance compounds employ higher levels of these compounds than the ones used in customary perfumery, these antibacterial compounds must meet several further criteria. They should have a relatively low odor value, i.e. they should only be perceived when used at higher amounts in order to not affecting the olfactive balance of the fragrance composition too much. The odor value is defined as the vapour pressure (in ng/L) divided by the olfactive threshold value (in ng/L) and it corresponds to the dilution factor above threshold (DFT). It was found, that commercial Sandela comprising all isomers has an odor value of 265, whereas Sandalore has an odor value of 5685, Ebanol has an odor value of 212557 and the odor value of Radjanol is 53130. Santalol, the main active ingredient of Sandalwood oil, has an odor value of 650 and Eugenol has even an odor value of 346904. Said compounds meet therefore the criterion of low odor value and their olfactive impact on the fragrance is much lower compared to other compounds with Sandalwood odor like Sandalore and Ebanol or other antibacterial perfume components like Eugenol. Thus they may be incorporated in quantities sufficiently high to obtain good antimicrobial activity in perfumes without affecting the overall impression too much. This would not be the case for other synthetic fragrance molecules with low threshold like Ebanol and Sandalore. Finally, their sandalwood note is highly desirable as a base note for perfumes applied to the human body.

2-methoxy-4-(5,5,6-trimethylbicyclo[2.2.1]hept-2-yl)cyclohexan-1-ol and 2-methoxy-5-(5,5,6-trimethyl-bicyclo[2.2.1]hept-2-yl)cyclohexan-1-ol are odorless and can therefore be included in cosmetic formulations without affecting the odor.

The expression perfume in the present invention stands for a mixture comprising odoriferous substances and other ingredients which are known to those skilled in the art. In a preferred embodiment the composition according to the present invention comprise a perfume, the latter comprising 10 to 80% by weight of the active component.

In an other preferred embodiment the composition according to the present invention comprises a perfume, the latter comprising 10 to 80% by weight of the active component as the only antibacterial agent.

The compositions according to the present invention need a active component level which is higher than the one used in customary perfumery. The recommended level in the final product should ideally be between 0.3% to 0.6%. If the perfume level in the deodorizing or antiperspirant product is kept at 1%, the perfume should contain between 30% and 60% of the active component. If the perfume level in the product is 1.5%, the perfume comprises preferably 20 to 40% of the active component. However, to obtain stronger effects the use level may be increased to 1% in the finished deodorant or antiperspirant product. Finally, if the active component is used in combination with other antibacterial fragrance materials the level in the final product may be lowered to 0.1%.

The active component in the composition according to the present invention does not need to be added to the perfume, but can also be added directly to the deodorant or antiperspirant product. In this case the active component is added separate from the perfume to the deodorant or antiperspirant formula at a level of 0.1 to 1%.

Furthermore it was tested whether the active component in the composition according to the present invention can be combined with other antibacterial agents currently used for their deodorizing action to reduce usage levels of these compounds or to improve their antibacterial effects. It was found that the active component does not enhance the activity of Triclosan, which may be due to a completely different mode of action of these two compounds. It appears therefore not useful to combine these two materials to improve the deodorant efficiency of a product.

In a preferred embodiment the composition according to the present invention comprise a perfume, the latter comprising 10 to 80% by weight of the active component.

In an other preferred embodiment the composition according to the present invention comprises a perfume, the latter comprising 10 to 80% by weight of the active component as the only antibacterial agent.

The active component can be used for partially replacing Farnesol in antibacterial fragrance compositions. Indeed, these two products can be combined in any ratio adding up their antibacterial effect. The invention thus further relates to the use of the active compound to reduce usage level of Farnesol. Due to the four times lower price of the active component compared to Farnesol, this results in significant cost savings and an improvement of the overall olfactive performance of the perfume. Like the active component, Farnesol can be added as an ingredient of the perfume or separately from the perfume. In antibacterial compositions comprising a perfume, the latter comprising the active component and Farnesol, the level of the active component is preferably between 10 and 80%, the level of Farnesol between 5 and 50%.

The active component according to the present invention cannot be employed only for cost savings and improved olfactive performance, it can be used to enhance the antibacterial effect of Farnesol as well. By keeping normal use levels of Farnesol, which is used as separate ingredient of the deodorant or antiperspirant product, and combining this with a perfume with high active component content, an enhanced antibacterial effect can be obtained. The invention thus also relates to the use of high levels of the active component as well as Farnesol in deodorant and antiperspirant products to obtain increased deodorant effects.

Compositions according to the present invention comprise beside one or more compound(s) of formula I at least one other ingredient. Such an ingredient may be water, dipropylene glycol or propylene glycol.

Compositions according to the present invention comprising the active component may be formulated in various forms such as deodorant stick, roll-on, pump-spray, aerosol, deodorant soap, powder, solution, gel, cream, stick, balm and lotion. These products are preferably used as personal care products.

### Examples

### Example 1.

### Comparison of the antibacterial activity of compound of the formula I with other perfume ingredients and antibacterial agents when tested against human axilla bacteria.

The antibacterial efficacy of the relevant fragrance compounds mentioned in the present invention in microtiter plate tests is demonstrated below. The different bacterial strains had been isolated from the human axilla by current microbiological practice. They were taxonomically identified by cell morphology, gram-reaction and biochemical tests included in the Api coryne test kit (BioMerieux, France). Strain *Staphylococcus epidermidis* Ax25 was identified by fatty acid methyl ester analysis (FAME; German type strain collection DSMZ, Germany). The strains were maintained on Tryptic soy broth plates, this standard medium being amended with 5 g per Litre of Tween 80 and 1 g of Soybean lecithin. Plates were incubated at 36° C for a period 48 hours. The bacteria were then swabbed from the plates and suspended in 4 ml of Müller-Hinton broth amended with 100 mg of Tween80 per Litre (MH-Tween) and incubated again at 36° C for 16 hours. Following incubation the bacterial suspensions were diluted in MH-Tween to obtain a final cell density of 10⁶ colony forming units per ml. For each fragrance material four microtiter plates were used in the example, each microtiter plate having 8 rows, A-H, and 12 columns, 1-12. To each column a diluted suspension of a different test organism was distributed, 100 µl per well. 1% stock solutions of the fragrance materials were then prepared in MH-Tween broth. The fragrance material was dispersed by ultrasonication into the aqueous medium to obtain a homogenous emulsion. 100 µl per well of this emulsion was then added to the first row of the microtiter plates already containing the target organisms. Serial dilution series were then prepared starting at row A and continuing until row G, each time removing 100 µl from a well and transferring it to the next well. In this way fragrance concentrations of 0.5%, 0.25%, 0.125%, 0.0625%, 0.03125%, 0.0156% and 0.0078% were tested for their antibacterial efficacy. The reference compound Triclosan was tested at 16 fold lower concentrations. The plates were covered with plastic films and incubated for 24 h at 36° C with shaking at 250 rpm. The turbidity developing in the microtiter plates was then examined after 24 h to determine microbial growth. The minimal concentration of fragrance inhibiting the growth of an organism by at least 80% was determined as the minimal inhibitory concentration (MIC).

**Table 1.**

| **Minimal inhibitory concentration (MIC) for bacteria derived from the human axilla of relevant perfume raw materials described in this study. Data are expressed in % weight/ volume** | | | | | | | | | | |
|---|---|---|---|---|---|---|---|---|---|---|
| | Ax 25 | Ax26 | Ax3 | Ax7 | Ax10 | Ax11 | Ax12 | Ax15 | Ax20 | Ax19 |
| Sandela | 0.0234 | 0.0156 | 0.0176 | 0.0273 | 0.0137 | 0.0273 | 0.0137 | 0.0234 | 0.0273 | 0.0086 |
| Farnesol | 0.0156 | 0.0078 | 0.0156 | 0.0137 | 0.0078 | 0.0195 | 0.0137 | 0.0156 | 0.0137 | 0.0156 |
| Sandal-wood oil | 0.0313 | 0.0156 | 0.0195 | 0.0313 | 0.0164 | 0.0313 | 0.0313 | 0.0313 | 0.0313 | 0.0313 |
| Sandalore | 0.1250 | 0.1250 | 0.1250 | 0.1250 | 0.1250 | 0.1250 | 0.1250 | 0.2500 | 0.1250 | n.d. |
| Radjanol | 0.1250 | 0.0625 | n.t. | 0.0625 | n.t. | n.t. | n.t. | 0.125. | n.t. | n.t. |
| Ebanol | 0.1250 | 0.1250 | 0.1250 | 0.1250 | 0.2500 | 0.2500 | 0.2500 | 0.2500 | 0.1250 | n.d. |
| Triclosan | 0.000015 | 0.0015 | 0.0029 | 0.0029 | 0.0020 | 0.0029 | 0.0017 | 0.0029 | 0.0107 | 0.0039 |

Ax 25 was identified as *Staphylococcus epidermidis* by FAME analysis. Identification with the Api Coryne test kit yielded the following species assignments for the remainder of the strains: Ax 26 *Corynebacterium sp.;* Ax 3 *Corynebacterium bovis;* Ax 7 *Corynebacterium group G;* Ax 10 *Corynebacterium jeikeium;* Ax 11 *Corynebacterium jeikeium;* Ax 12 *Corynebacterium jeikeium;* Ax 15 *Corynebacterium jeikeium;* Ax 20 *Corynebacterium striatum/amycolatum;* Ax 19 *Corynebacterium jeikeium.* The strains were isolated from the axilla of 8 human volunteers. Based on biochemical tests and colony morphology all strains were distinct from each other, even the several strains all belonging to *Corynebacterium jeikeium*.

### Example 2. Antibacterial activity against Brevibacterium epidermidis

A selection of perfume compounds were tested against Brevibacterium epidermidis DSMZ 9586 (German Collection of Microorganisms and Cell Cultures) with the method described in Example 1. The MIC value obtained for Sandela is comparable with the values reported for the axilla bacteria, and it is thus appropriate to use this perfume compound also in deodorant products intended to prevent the cheesy smell of feet and shoes.

| MIC (% w/v) for *Brevibacterium epidermidis* | |
|---|---|
| Sandela | 0.0156 |
| Sandalore | 0.0937 |
| Sandalwood oil | 0.0156 |
| Radjanol | 0.03125 |
| Farnesol | 0.0078 |
| Triclosan | 0.003125 |

### Example 3. Antibacterial activity against Propionibacterium acnes

A selection of perfume compounds were tested against Propionibacterium acnes DSMZ1897 (German Collection of Microorganisms and Cell Cultures) with the method described in Example 1, with the exception that the organism was grown under anaerobic conditions during 72 hours until evaluation of results.

| MIC (% w/v) for *Propionibacterium acnes* | |
|---|---|
| Sandela | 0.03125 |
| Sandalore | 0.25 |
| Sandalwood oil | 0.0156 |
| Farnesol | 0.0078 |
| Triclosan | 0.00039 |

### Example 4. Comparison of the antibacterial action of perfume oils with high Sandela and Farnesol content

Perfume compositions with high Sandela and/or Farnesol content were prepared as described in Table 2. These are typical perfumes to be incorporated in cosmetic products with deodorant or antiperspirant action. The MIC of these perfume compositions against a selection of the test organisms was tested with the method described in Example 1. The results are summarised in Table 3. Indeed these perfumes have a MIC between 0.025% and 0.05% which indicates strong antibacterial activity. Furthermore it becomes evident from these data, that Farnesol can be partially or fully replaced with Sandela in a perfume composition without loosing the activity significantly. Finally, adding Farnesol in addition to Sandela further enhances the activity of such compositions.

**Table 2.**

| **Composition of deodorant perfumes (parts per weight)** | | | | |
|---|---|---|---|---|
| | composition | | | |
| | A | B | C | D |
| ACET CEDRENYL 70 | 60 | 60 | 60 | 43 |
| ACET LINALYLE SYNT | 30 | 30 | 30 | 21 |
| ACET P T BUTYL CYCLOHEXYLE | 55 | 55 | 55 | 39 |
| ADOXAL at 10% in DPG | 2 | 2 | 2 | 1 |
| ALD PHENYL ACETIQUE 85%/APE | 10 | 10 | 10 | 7 |
| ALLYL AMYL GLYCOLATE | 12 | 12 | 12 | 9 |
| AMBROFIX at 10% in DPG | 4 | 4 | 4 | 3 |
| BRASSYLATE ETHYLENE | 20 | 20 | 20 | 14 |
| CEDRYL METHYL ETHER | 20 | 20 | 20 | 14 |
| CITRAL SYNT 80%/ORANGE TERPENES | 1 | 1 | 1 | 1 |
| DIHYDRO MYRCENOL | 130 | 130 | 130 | 93 |
| EVERNYL | 1 | 1 | 1 | 1 |
| FARNESOL | 400 | 0 | 200 | 200 |
| GERANIOL | 20 | 20 | 20 | 14 |
| GERANIUM ESS AFRIQUE | 20 | 20 | 20 | 14 |
| ISO E SUPER | 100 | 100 | 100 | 71 |
| LILIAL | 120 | 120 | 120 | 86 |
| LINALOL SYNT | 50 | 50 | 50 | 37 |
| SALICYLATE HEXENYLE-3-CIS | 15 | 15 | 15 | 11 |
| SANDELA | 0 | 400 | 200 | 400 |
| TROPIONAL | 30 | 30 | 30 | 21 |
| Total | 1100 | 1100 | 1100 | 1100 |

**Table 3.**

| **Antibacterial activity of deodorant perfumes** | | | | |
|---|---|---|---|---|
| | composition | | | |
| | A | B | C | D |
| Ax25 | 0.0625 | 0.0625 | 0.0625 | 0.03125 |
| S. epidermidis | | | | |
| Ax26 | 0.02343 | 0.0625 | 0.0117 | 0.0156 |
| Corynebacterium xerosis | | | | |
| Ax7 | 0.03125 | 0.03125 | 0.03125 | 0.03125 |
| Corynebacterium group G | | | | |
| Ax15 | 0.03125 | 0.03125 | 0.03125 | 0.03125 |
| Corynebacterium jeikeium | | | | |
| average | 0.037108 | 0.046875 | 0.034175 | 0.027338 |

1) Concentration in percent (w/v) needed for at least 80% reduction in growth during 24 hours.

### Example 5. Antibacterial activity of Deodorant Roll-on formula incorporating antibacterial perfumes

Non-alcoholic deodorant compositions for Roll-on deodorants were prepared as described in Table 4. The Deodorants were ammended with 1.4% of the perfumes according to the present invention which were described in Example 4. The antibacterial activity of the deodorants was determined according to the procedure described in Example 1. Instead of using emulsions of the perfume products in MH-Tween, 100 µl of the finished deodorant was added to the first well of the Microtiter plates containing the target organisms. Dilution series of this finished product were thus prepared in MH-Tween, and growth of the microorganisms in these product solutions were monitored.

**Table 4.**

| **Composition of Deodorants containing perfumes according the present invention** | |
|---|---|
| Hydroxyethylcellulose | 0.30 |
| Water | 62.1 |
| Dipropylene Glycol | 30.00 |
| Propylene Glycol | 5.00 |
| PEG 40 hydrogenated Castor oil | 2.50 |
| Tetrasodium EDTA | 0.10 |
| Citric acid | qsp pH= 5.50 |
| Fragrance | 1.4 |

Hydroxyethylcellulose was dispersed in water at 35°C till homogeneous. PEG 40 hydrogenated Castor oil was mixed with dipropylene glycol and propylene glycol and this mixture was added. The remaining constituants were added afterwards.

**Table 5.**

| **Growth inhibitory effect of the Deodorant formula according to table 4 with the perfume compositions described in Example 4.** | | | | |
|---|---|---|---|---|
| | no | 1.4% | 1.4% | 1.4% |
| | perfume | perfume | perfume | perfume |
| | | B | C | A |
| Staphylococcus | | | | |
| epidermidis Ax 25 | 11 | 38 | 32 | 64 |
| Corynebacterium | | | | |
| xerosis Ax 26 | 10 | 45 | 45 | 128 |
| Corynebacterium | | | | |
| group G Ax10 | 27 | 54 | 54 | 128 |
| Corynebacterium | | | | |
| jeikeium Ax 15 | 27 | 54 | 32 | 64 |

Given is the maximal dilution of the product which still inhibits the growth of the axilla bacteria

### Example 6:Further examples of deodorants

The following sets forth examples for the use of perfume compositions according to the present invention in various products. The methods of preparing the following compositions are well known to those skilled in the art. All formulations may contain additional ingredients known to those skilled in the art, e.g. colorants, opacifiers, buffers, antioxidants, vitamins, emulsifiers, UV absorbers, silicones and the like. All products can also be buffered to the desired pH. All values are % w/w.

| **A) Deo-colognes:** | |
|---|---|
| Fragrance | 0.5 - 10.0 |
| Ethanol | to 100.0 |

| **B) Deo-Sticks:** | |
|---|---|
| **- Antiperspirant stick:** | |
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminium Sesquichlorhydrate | 20.0 |
| Fragrance | 1.0 |

| **- Clear Deodorant Stick** | |
|---|---|
| Witconol APM | 43.0 |
| Propylene Glycol | 20.0 |
| Alcohol 39C | 21.0 |
| Water | 7.0 |
| Monamid 150ADD | 5.0 |
| Millithix 925 | 2.0 |
| Ottasept Extra | 0.5 |
| Fragrance | 1.5 |

| **- Antiperspirant Aerosol** | |
|---|---|
| Absolute Ethanol | 15.0 |
| Zirconium Aluminum tetrachlorhydrate | 5.0 |
| Bentone 38 | 1.5 |
| Fragrance | 1.25 |
| S-31 Hydrocarbon propellant | to 100.0 |

| **- Roll-On** | |
|---|---|
| Dimethicone DC 354 (Dow Corning) | 69.0 |
| Bentone 38 | 10.0 |
| Rezal 36 GP (Reheis Chem. Co.) | 20.0 |
| Fragrance | 1.0 |

In all these compositions, the fragrance contains from 10-60% of Sandela and from 0 -50% of Farnesol.

In the above, the following components were used:

| | |
|---|---|
| Neobee 1053 | glycerol tricaprate/caprylate |
| Generol 122 | soya sterol |
| Kesscowax B | cetyl alcohol and glycol polymer |
| Witconol APM | polypropylene glycol-3 myristyl ether |
| Monamid 150ADD | cocoamide diethanolamine |
| Millithix 925 | dibenzylidene sorbitol |
| Ottasept Extra | quaternium 18 hectorite |
| Bentone 38 | quaternium 18 hectorite |
| Dimethicone DC 354 | mixture of fully methylated linear siloxane polymers end-blocked with trimethylsiloxy units |
| Rezal 36 GP | aluminium zirconiumtetrachlorohydrexglycine |

### Example 7. Comparison of the antibacterial activity of sandela distillation fractions with varying content of different sandela isomers

Since sandela is known to be a mixture of different reaction products, commercial sandela was subjected to a distillation process. A total of 16 fractions were obtained in a distillation of 2350 g commercial sandela over a 1.3 m column at a pressure of 0.1 Torr. The different fractions were evaluated for their antimicrobial activity (Table 6) with the method described in example 1. From these results it appears, that the late fractions 14-15 are more active than the early fraction 2-3. Even more active was the last fraction 16, which obviously contains the most active compounds. GC-MS analysis of the single fractions led to the conclusion, that fractions 14-15 contain different isomers of 3-(5,5,6-trimethylbicyclo[2.2.1]hept-2-yl)-cyclohexanol and 4-(5,5,6-trimethylbicyclo[2.2.1]hept-2-yl)-cyclohexanol whilst fraction 2-3 have a high content of 2-(5,5,6-trimethylbicyclo[2.2.1]hept-2-yl)-cyclohexanol. The main peaks in fraction 16 could be attributed to neither of the above mentioned isomers.

**Table 6.**

| **Minimal inhibitory concentration (MIC) for different fractions obtained from sandela by distillation (data are expressed in % weight/ volume)** | | | |
|---|---|---|---|
| | Boiling point (°C) | S. epidermidis | Corynebacteria (average for three organisms) |
| sandela | | 0.023 | 0.022 |
| Fraction 1-2 | 109-119_{0.1 Torr} | 0.063 | 0.077 |
| Fraction 14-15 | 131_{0.08 Torr} | 0.022 | 0.022 |
| Fraction 16 | >131_{0.08 Torr} | 0.008 | 0.008 |

### Example 8. Isolation and structure elucidation of compounds from most active distillation fraction of sandela

As can be seen from Example 7, the latest fraction (16) of the distillation process contains the most active products. The main peaks in the gas chromatogram within this sample were subjected to GC-MS analysis and it was found that they do not correspond to sandela isomers but that their mass spectrum would conform to compounds having an additional methoxygroup (=methoxysandela): 2-methoxy-4-(5,5,6-trimethylbicyclo[2.2.1]hept-2-yl)-cyclohexanol and 2-methoxy-5-(5,5,6-trimethylbicyclo[2.2.1]hept-2-yl)-cyclohexanol. This sample was therefore again subjected to a fractional distillation and the single fractions were examined for antibacterial activity and compared with GC-MS analysis. Fractions with increased methoxysandela content had the highest activity.

Therefore the fraction having both the highest activity and the highest content of methoxysandela isomers was subjected to column chromatography with silica resin (0.063 - 0.2 mm) using hexane / methyl-tert-butyl ether (1:1) as eluent. Chromatography fractions which contained only methoxysandela isomers were pooled. The resulting sample still contained three peaks when evaluated with gas chromatography. These peaks were separated with preparative gas chromatography and the structure was elucidated with NMR. The spectra conformed to the following structures:

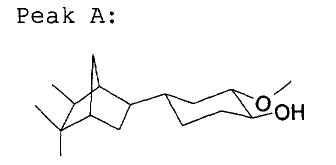

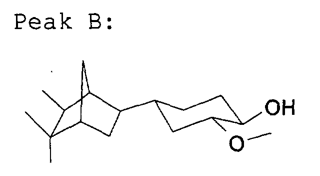

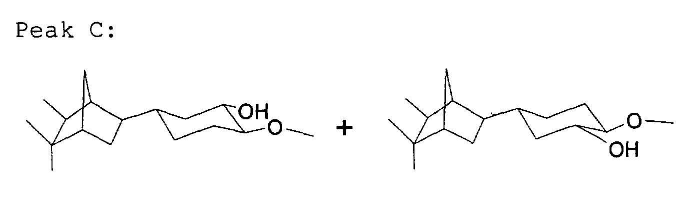

Finally, the different isomers obtained by preparative GC and some distillation and chromatography fractions from the separation procedure were compared for their minimal inhibitory concentration as described in example 1. From the results shown in Table 7 it can be seen that the most active isomers have an antimicrobial activity which is comparable to triclosan when measured against the malodor forming bacteria colonizing the human skin.

**Table 7.**

| **Bacteriostatic activity for distillation fractions and pure compounds obtained from sandela compared to benchmarks** | | | | |
|---|---|---|---|---|
| | S. epidermidis | Corynebact. Ax26 | Corynebact. Ax7 | Corynebact. Ax15 |
| sandela | 0.023 | 0.0156 | 0.0273 | 0.0234 |
| Fraction 16 | 0.0131 | 0.0086 | 0.0156 | 0.0130 |
| Mixture of Peak A, B and C * | 0.0083 | 0.0048 | 0.0083 | 0.0083 |
| Peak A | 0.0040 | 0.0028 | 0.0048 | 0.0048 |
| Peak B | 0.0023 | 0.0016 | 0.0028 | 0.0028 |
| Peak C | 0.0083 | 0.0048 | 0.0083 | 0.0083 |
| Farnesol | 0.0156 | 0.0078 | 0.0137 | 0.0156 |
| Triclosan | 0.000015 | 0.0025 | 0.0025 | 0.0030 |

| | | | | |
|---|---|---|---|---|
| *obtained by column chromatography | | | | |

1) concentration in percent (w/v) needed for at least 80% reduction in growth during 24 hours.

### Example 9. Comparision of activity of different isomers of methoxysandela.

The different isomers obtained by preparative GC and some distillation and chromatography fractions from the separation procedure outlined in example B were compared for their minimal inhibitory concentration as described in example 1. From the results shown in Table Z it can be seen that the most active isomers have an antimicrobial activity which is comparable to triclosan when measured against the malodor forming bacteria colonizing the human skin.

**Table 8.**

| **Bacteriostatic activity for distillation fractions and pure compounds obtained from sandela compared to benchmarks** | | | | |
|---|---|---|---|---|
| | S. epidermidis | Coryneba ct. Ax26 | Coryneba ct. Ax7 | Coryneba ct. Ax15 |
| sandela | 0.023 | 0.0156 | 0.0273 | 0.0234 |
| Fraction 16 | 0.0131 | 0.0086 | 0.0156 | 0.0130 |
| Mixture of Peak A, B and C ob-tained by column chromatography | 0.0083 | 0.0048 | 0.0083 | 0.0083 |
| Peak A | 0.0040 | 0.0028 | 0.0048 | 0.0048 |
| Peak B | 0.0023 | 0.0016 | 0.0028 | 0.0028 |
| Peak C | 0.0083 | 0.0048 | 0.0083 | 0.0083 |
| Farnesol | 0.0156 | 0.0078 | 0.0137 | 0.0156 |
| Triclosan | 0.000015 | 0.0025 | 0.0025 | 0.0030 |

1)concentration in percent (w/v) needed for at least 80% reduction in growth during 24 hours.

## Claims

1. An antibacterial composition comprising one or more compound(s) of formula I 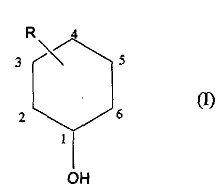 as an active component, and at least one other ingredient,
wherein formula I R is a residue of the formula II 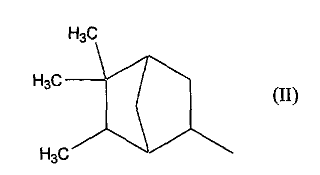 at the position 2, 3, 4 or 5 and
if R is at the position 5, a hydrogen in position 2 is substituted by a methoxy group, and
if R is at the position 4, a hydrogen in position 2 may optionally be substituted by a methoxy group.

2. Composition according to claim 1, wherein the active component is 2-methoxy-4-(5,5,6-trimethyl-bicyclo[2.2.1]hept-2-yl)cyclohexan-1-ol.

3. Composition according to claim 1, wherein the active component is 3-(5,5,6-trimethylbicyclo[2.2.1]hept-2-yl)cyclohexan-1-ol).

4. Composition according to claim 1 or 2, comprising 0.1 to 1% by weight of the active component.

5. Composition according to claim 1 or 2, comprising 0.3 to 0.6% by weight of the active component.

6. Composition according to one of the claims 1 to 5 comprising additionally 3,7,11-trimethyl-2,6,10-dodecatrien-1-ol.

7. Composition according to one of the claims 1 to 6 comprising a perfume, the latter comprising 10 to 80% by weight of the active component.

8. Composition according to one of the claims 1 to 7 comprising a perfume, the latter comprising 10 to 80% by weight of the active component as the only antibacterial agent.

9. Composition according to one of the claims 1 to 8 comprising a perfume, the latter comprising 10 to 80% by weight of the active component and 5 to 50 % of 3,7,11-trimethyl-2,6,10-dodecatrien-1-ol.

10. A personal care product comprising a composition according to one of the claims 1 to 9.

11. A malodor inhibiting product according to claim 10.

12. An acne inhibiting product according to claim 10.

13. A deodorant and/or an antiperspirant product according to claim 10.

14. Method for preparing a product according to claim 10 by admixing all ingredients and adding the active component together with fragrance components.

15. Method for preparing a product according to claim 10 by admixing all ingredients, adding active component together with fragrance components and adding 3,7,11-trimethyl-2,6,10-dodecatrien-1-ol separately.

16. Method for preparing a product according to claim 10 by admixing all ingredients, adding active component separately from fragrance components.
